# EUROPEAN PATENT APPLICATION

(11) **EP 4 671 556 A1**
(43) Date of publication of application: **31.12.2025**
(21) Application number: 24780711.8
(22) Date of filing: 28.03.2024
(51) Int. Cl.: F16D 11/00, A61F 2/68, F16D 15/00, F16D 21/00, F16H 1/06, F16H 1/16, F16H 1/32, F16H 3/70

(54) **POWER TRANSMISSION DEVICE AND JOINT DEVICE**

(30) Priority: 31.03.2023 JP 2023058069
(71) Applicant: HONDA MOTOR CO., LTD., Tokyo 105-8404 (JP)
(72) Inventor: SHIMADA, Kei, Wako-shi, Saitama 351-0193 (JP); ONO, Hiromi, Wako-shi, Saitama 351-0193 (JP)
(74) Representative: Kiwit, Benedikt
(86) International application number: PCT/JP2024/012822
(87) International publication number: WO 2024/204612

(57) **Abstract**

A power transmission device (TM1) includes a connection and disconnection mechanism (20) and a transmission (T). The transmission (T) includes a second planetary mechanism (P2) having a sun gear (SG2), a carrier (PC2), and a ring gear (RG2). The transmission (T) changes in speed of rotation power between a first shaft (181) and a second shaft (182). The carrier (PC2) and the ring gear (RG2) are disposed outward than the second shaft (182) in a radial direction. At least a part of the carrier (PC2) and the ring gear (RG2) overlap the second shaft (182) as viewed in the radial direction.

## Description

### TECHNICAL FIELD

The present invention relates to a power transmission device and a joint device.

### BACKGROUND ART

In the related art, a power transmission device is known which includes a transmission that speed-changes power of a power source and a connection and disconnection mechanism that switches between disconnection and connection of the power. For example, Patent Literature 1 discloses that such a power transmission device is used for a joint device such as an electric prosthetic leg. In the joint device described in Patent Literature 1, the transmission has two power transmission paths having different transmission ratios, and the power transmission paths are switched between a stance and a swing.

### CITATION LIST

### PATENT LITERATURE

Patent Literature 1: WO2021/251500A

### SUMMARY OF INVENTION

### TECHNICAL PROBLEM

The power transmission device is desired to be small in size because the power transmission device is used in various devices.

The present invention provides a power transmission device and a joint device that enable to be reduced in size.

### SOLUTION TO PROBLEM

The present invention is a power transmission device including:
a connection and disconnection mechanism; and
a transmission, in which
the connection and disconnection mechanism includes:
   an engagement element disposed between a first rotation member and a second rotation member; and
   an operation unit configured to operate the engagement element to an engagement state where the first rotation member and the second rotation member are integrally rotatable and a non-engagement state where the first rotation member and the second rotation member are relatively rotatable,
the operation unit includes an actuation element configured to move the engagement element, and an operation element provided to be capable of operating the engagement element via the actuation element or capable of operating the engagement element not via the actuation element,
the first rotation member and the second rotation member are disposed such that respective rotation axes of the first rotation member and the second rotation member coincide with each other, and at least a part of the first rotation member and the second rotation member overlap each other as viewed in a radial direction with respect to the rotation axes,
the operation element includes:
   an advance and retraction element provided to be capable of advancing and retracting along the radial direction; and
   an extending portion extending along the rotation axes and provided to be capable of advancing and retracting along the rotation axes,
the transmission includes a transmission unit including:
   a first rotation element configured by the first rotation member or a rotation member rotating integrally with the first rotation member;
   a second rotation element provided to be capable of transmitting rotation with the first rotation element; and
   a third rotation element provided to be capable of transmitting rotation with the second rotation element,
the second rotation element and the third rotation element are:
   disposed outward than the second rotation member in the radial direction; and
   disposed such that at least a part of the second rotation element and the third rotation element overlaps the second rotation member as viewed in the radial direction.

Further, the present invention is a power transmission device including:
a connection and disconnection mechanism; and
a transmission, in which
the connection and disconnection mechanism includes:
   an engagement element disposed between a first rotation member and a second rotation member; and
   an operation unit configured to operate the engagement element to an engagement state where the first rotation member and the second rotation member are integrally rotatable and a non-engagement state where the first rotation member and the second rotation member are relatively rotatable,
the operation unit includes an actuation element configured to move the engagement element, and an operation element provided to be capable of operating the engagement element via the actuation element or capable of operating the engagement element not via the actuation element,
the first rotation member and the second rotation member are disposed such that respective rotation axes of the first rotation member and the second rotation member coincide with each other, and at least a part of the first rotation member and the second rotation member overlap each other as viewed in a radial direction with respect to the rotation axes,
the operation element includes:
   an advance and retraction element provided to be capable of advancing and retracting along the radial direction; and
   an extending portion extending along the rotation axes and provided to be capable of advancing and retracting along the rotation axes,
the transmission includes a planetary transmission unit including:
   a first rotation element;
   a third rotation element;
   a fourth rotation element disposed to be capable of transmitting power between the first rotation element and the third rotation element; and
   a second rotation element supporting the fourth rotation element in a rotatable and revolvable manner,
the planetary transmission unit is configured such that rotation speeds of three rotation elements including the first rotation element, the third rotation element and the second rotation element satisfy a collinear relationship in which the rotation speeds are aligned on a single straight line in a collinear diagram, and
in the planetary transmission unit, a rotation axis of the third rotation element corresponds to the rotation axes of the first rotation member and the second rotation member.

Further, the present invention is a joint device including:
a first member;
a second member;
a coupling unit configured to couple the first member and the second member such that a formed angle formed between the first member and the second member is variable; and
an enlarging and reducing device capable of enlarging and reducing the formed angle formed between the first member and the second member, in which
the enlarging and reducing device includes the power transmission device described above.

Further, the present invention is a joint device including:
a first member;
a second member;
a coupling unit configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, and a power transmission device configured to transmit power of the power source,
the power transmission device has:
   a first power transmission path for transmitting the power at a first transmission ratio; and
   a second power transmission path for transmitting the power at a second transmission ratio different from the first transmission ratio,
the enlarging and reducing device includes:
   a first connection and disconnection mechanism configured to switch between disconnection and connection of power in the first power transmission path; and
   a second connection and disconnection mechanism configured to switch between disconnection and connection of power in the second power transmission path,
the first power transmission path includes a planetary transmission unit, and
the second power transmission path includes the planetary transmission unit and another planetary transmission unit different from the planetary transmission unit.

### ADVANTAGEOUS EFFECTS OF INVENTION

According to the present invention, it is possible to provide the power transmission device and the joint device that enable to be reduced in size.

### BRIEF DESCRIPTION OF DRAWINGS

[FIG. 1] FIG. 1 is a cross-sectional view of a power transmission device TM1 according to a first embodiment of the present invention.
[FIG. 2] FIG. 2 is a cross-sectional view taken along a line X-X in FIG. 1.
[FIG. 3] FIG. 3 is a cross-sectional view taken along a line Y-Y in FIG. 1.
[FIG. 4] FIG. 4 is a perspective view of a retainer 282.
[FIG. 5] FIG. 5 is a cross-sectional view showing that a connection and disconnection unit 212 of a first connection and disconnection mechanism 210 is in an off state.
[FIG. 6] FIG. 6 is a cross-sectional view showing that the connection and disconnection unit 212 of the first connection and disconnection mechanism 210 is in an on state.
[FIG. 7] FIG. 7 is a diagram showing operations of the power transmission device TM1, in which (A) in FIG. 7 is a cross-sectional view showing the power transmission device TM1 in a neutral state, (B) in FIG. 7 is a cross-sectional view showing the power transmission device TM1 in a high-speed rotation state, and (C) in FIG. 7 is a cross-sectional view showing the power transmission device TM1 in a high torque state.
[FIG. 8] FIG. 8 is a cross-sectional view showing a power transmission device TM2 according to a second embodiment of the present invention.
[FIG. 9] FIG. 9 is a cross-sectional view showing a power transmission device TM3 according to a third embodiment of the present invention.
[FIG. 10] FIG. 10 is a cross-sectional view showing a power transmission device TM4 according to a fourth embodiment of the present invention.
[FIG. 11] FIG. 11 is a cross-sectional view showing the power transmission device TM4 according to a modification of the fourth embodiment of the present invention.
[FIG. 12] FIG. 12 is a diagram showing a fifth embodiment of the present invention and is a perspective view of an electric prosthetic leg 1 in which the power transmission device TM1 is assembled.
[FIG. 13] FIG. 13 is a side view of the electric prosthetic leg 1 in FIG. 12.
[FIG. 14] FIG. 14 is a view showing an internal structure of the electric prosthetic leg 1 in FIG. 12.
[FIG. 15] FIG. 15 is a diagram showing actions (stair ascending actions) of a user and the electric prosthetic leg 1 when ascending stairs.
[FIG. 16] FIG. 16 is a diagram showing actions (flat ground walking actions) of the user and the electric prosthetic leg 1 when walking on a flat ground.
[FIG. 17] FIG. 17 is a diagram showing a sixth embodiment of the present invention and is a diagram showing an internal structure of the electric prosthetic leg 1 in which the power transmission device TM1 is assembled.
[FIG. 18] FIG. 18 is a diagram showing a seventh embodiment of the present invention and is a diagram showing a configuration of a moving object M1 in which the power transmission device TM1 is assembled.

### DESCRIPTION OF EMBODIMENTS

Hereinafter, a power transmission device according to each embodiment of the present invention will be described with reference to the drawings.

### (First Embodiment)

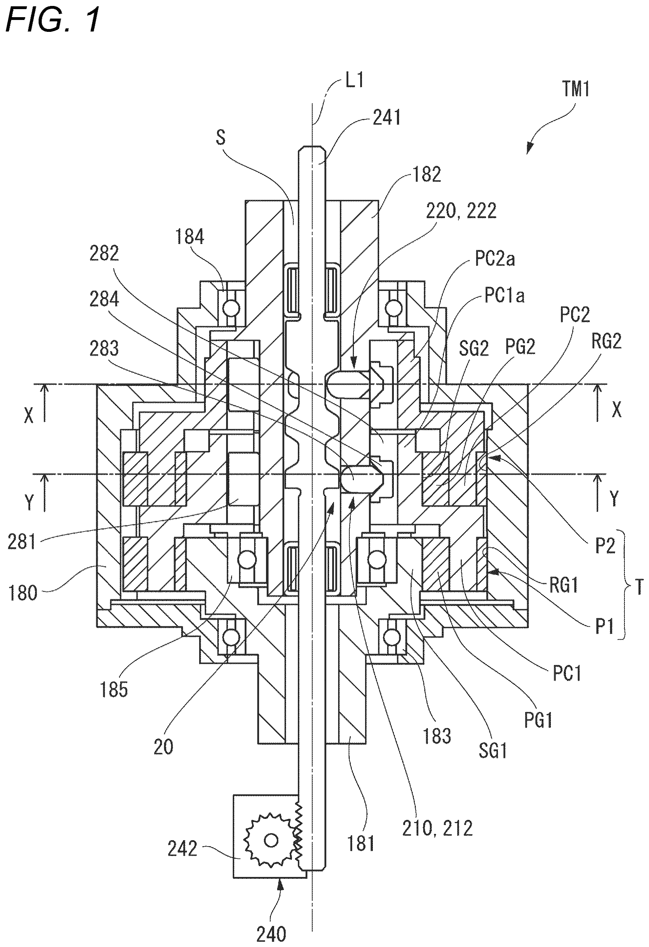

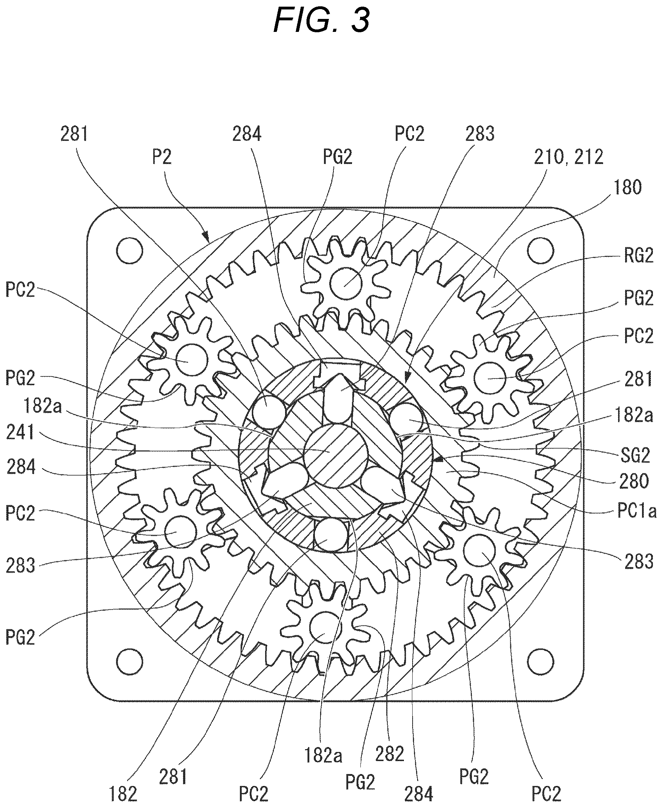

As shown in FIGS. 1 to 3, a power transmission device TM1 includes a first shaft 181 and a second shaft 182 having the same rotation axis (hereinafter, referred to as a rotation axis L1), a connection and disconnection mechanism 20 and a transmission T which are provided on a power transmission path between the first shaft 181 and the second shaft 182, and a case 180 that accommodates these parts. The case 180 has a substantially columnar shape, rotatably supports the first shaft 181 via a bearing 183, and rotatably supports the second shaft 182 via a bearing 184. A tip end portion of the first shaft 181 is exposed from one end side (lower side in FIG. 1) of the case 180 in a rotation axis direction, and a tip end portion of the second shaft 182 is exposed from the other end side (upper side in FIG. 1) of the case 180 in the rotation axis direction. In first to sixth embodiments, a case where the power transmission devices TM1 to TM4 are arranged disposed such that the rotation axis direction is an upper-lower direction will be described as an example. Hereinafter, in descriptions of the power transmission devices TM1 to TM4 of the first to sixth embodiments, the rotation axis direction is also referred to as the upper-lower direction.

The transmission T constitutes a first power transmission path that transmits power of a power source (not shown) received from the first shaft 181 to the second shaft 182 at a first transmission ratio, and a second power transmission path that transmits the power to the second shaft 182 at a second transmission ratio different from the first transmission ratio. As will be described in detail below, the transmission T includes a first planetary mechanism P1 and a second planetary mechanism P2. The first power transmission path includes the first planetary mechanism P1, and the second power transmission path includes the first planetary mechanism P1 and the second planetary mechanism P2.

The connection and disconnection mechanism 20 includes a first connection and disconnection mechanism 210 and a second connection and disconnection mechanism 220. As will be described in detail later, each of the first connection and disconnection mechanism 210 and the second connection and disconnection mechanism 220 switches between disconnection and connection of the power in the first power transmission path and switches between disconnection and connection of the power in the second power transmission path.

The first planetary mechanism P1 and the second planetary mechanism P2 respectively include sun gears SG1, SG2, ring gears RG1, RG2, a plurality of planetary gears PG1 and PG2 that mesh with the sun gears SG1, SG2 and the ring gears RG1, RG2, and carriers PC1, PC2 that support the planetary gears PG1, PG2 in a rotatable and revolvable manner. The three rotation elements, including the sun gears SG1, SG2, the ring gears RG1, RG2, and the carriers PC1, PC2 satisfy a collinear relationship in which respective rotation speeds are always aligned on a single straight line in a velocity collinear diagram (also referred to as a collinear diagram).

The sun gears SG1, SG2 and the carriers PC1, PC2 of the first planetary mechanism P1 and the second planetary mechanism P2 have the rotation axis L1 the same as those of the first shaft 181 and the second shaft 182. The first planetary mechanism P1 and the second planetary mechanism P2 are aligned in the rotation axis direction (upper-lower direction). The second planetary mechanism P2 is disposed on an outer side of the second shaft 182 and at least partially overlaps the second shaft 182 as viewed in a radial direction. In the present embodiment, the first planetary mechanism P1 is disposed below the second planetary mechanism P2, the first planetary mechanism P1 is also disposed on the outer side of the second shaft 182, and at least partially overlaps the second shaft 182 as viewed in the radial direction.

The sun gear SG1 of the first planetary mechanism P1 is formed integrally with the first shaft 181. The sun gear SG1 of the first planetary mechanism P1 does not necessarily have to be formed integrally with the first shaft 181, and may be formed separately from the first shaft 181 and connected to the first shaft 181 in an integrally rotatable manner. Thus, rotation power of the first shaft 181 is input to the first planetary mechanism P1. The sun gear SG1 of the first planetary mechanism P1 is relatively rotatably supported by the second shaft 182 via a bearing 185 disposed on an inner side of the sun gear SG1.

The ring gear RG1 of the first planetary mechanism P1 is formed integrally with the case 180. That is, the ring gear RG1 is not rotatable. The ring gear RG1 of the first planetary mechanism P1 does not necessarily have to be formed integrally with the case 180, and may be formed separately from the case 180 and mechanically connected to the case 180 in a non-rotatable manner.

Thus, the carrier PC1 of the first planetary mechanism P1 decelerates and outputs the rotation received from the sun gear SG1.

The carrier PC 1 of the first planetary mechanism P1 is formed integrally with the sun gear SG2 of the second planetary mechanism P2. In the present embodiment, the sun gear SG2 is formed on an outer peripheral portion of a tubular portion PC1a integrally formed with the carrier PC1. Thus, the rotation power decelerated by the first planetary mechanism P1 can be transmitted to the second planetary mechanism P2.

The sun gear SG2 (tubular portion PC1a) of the second planetary mechanism P2 is connected to the second shaft 182 via the first connection and disconnection mechanism 210. That is, in the present embodiment, the first connection and disconnection mechanism 210 is provided between the tubular portion PC1a integrally formed with the carrier PC1 and the second shaft 182 located on an inner peripheral portion of the tubular portion PC1a. Thus, the first power transmission path including the first planetary mechanism P1 is formed.

The ring gear RG2 of the second planetary mechanism P2 is formed integrally with the case 180. That is, the ring gear RG2 is not rotatable. The ring gear RG2 of the second planetary mechanism P2 does not necessarily have to be formed integrally with the case 180, and may be formed separately from the case 180 and mechanically connected to the case 180 in a non-rotatable manner.

Thus, the carrier PC2 of the second planetary mechanism P2 decelerates and outputs the rotation received from the sun gear SG2.

The carrier PC2 of the second planetary mechanism P2 is connected to the second shaft 182 via the second connection and disconnection mechanism 220. In the present embodiment, the second connection and disconnection mechanism 220 is provided between the tubular portion PC2a integrally formed with the carrier PC2 and the second shaft 182 located on an inner peripheral portion of the tubular portion PC2a. The tubular portion PC2a does not necessarily have to be formed integrally with the carrier PC2, and may be formed separately from the carrier PC2 and mechanically connected to the carrier PC2 in an integrally rotatable manner. Although not shown, bearings that allow relative rotation may be provided between the rotation elements of the first planetary mechanism P1 and the rotation elements of the second planetary mechanism P2, and between the case 180 and the rotation elements of the first planetary mechanism P1 or the second planetary mechanism P2.

In the transmission T configured in this way, the first power transmission path for transmitting the power of the power source received from the first shaft 181 to the second shaft 182 via the first planetary mechanism P1 and the first connection and disconnection mechanism 210 is formed, and the second power transmission path for transmitting the power to the second shaft 182 via the first planetary mechanism P1, the second planetary mechanism P2, and the second connection and disconnection mechanism 220 is formed.

The first connection and disconnection mechanism 210 includes a connection and disconnection unit 212 provided between the tubular portion PC1a and the second shaft 182. The second connection and disconnection mechanism 220 includes a connection and disconnection unit 222 provided between the tubular portion PC2a and the second shaft 182. Details of the connection and disconnection units 212, 222 will be described below with reference to FIGS. 1 to 6.

The connection and disconnection units 212, 222 have a common configuration and are configured to be switched between a disconnection state where the power transmission path is disconnected and a connection state where the power transmission path is connected. Each of the connection and disconnection units 212, 222 of the present embodiment is configured using a two-way clutch 280 with a forced free function. The two-way clutch 280 includes a plurality of (three in the present embodiments) rollers 281 that are arranged between an outer peripheral surface portion of the second shaft 182 and inner peripheral surface portions of the tubular portions PC1a, PC2a, a retainer 282 that holds the plurality of rollers 281 at predetermined intervals, an operation mechanism 240, a plurality of (three in the present embodiment) pins 283 that penetrate the second shaft 182 in the radial direction and are operated by the operation mechanism 240 to a forced free position and a forced free release position, and a plurality of (three in the present embodiment) guides 284 that are provided in the retainer 282 and defines a relative rotation position of the retainer 282 with respect to the second shaft 182 when the pins 283 are at the forced free position. The rollers 281 may be balls or sprags.

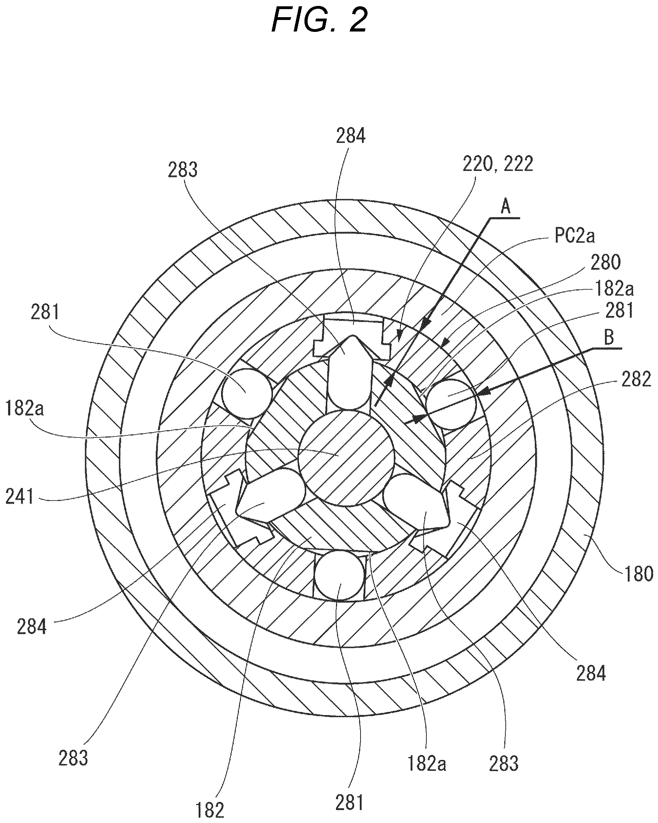

As shown in FIG. 2, a distance A in the radial direction between the outer peripheral surface portion of the second shaft 182 and the inner peripheral surface portions of the tubular portions PC1a, PC2a is smaller than a diameter B of the roller 281. Further, flat portions 182a are formed on the outer peripheral portion of the second shaft 182 at predetermined intervals in a circumferential direction, and on a center side in the circumferential direction of each of the flat portions 182a, the distance A is larger than the diameter B.

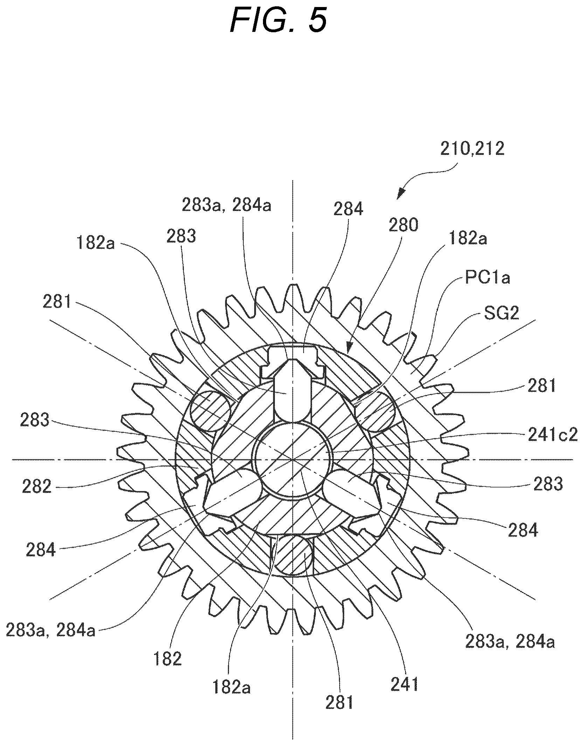

In other words, in a state where the rollers 281 are held at center portions of the flat portions 182a in the circumferential direction, the rollers 281 do not mesh with the outer peripheral surface portion of the second shaft 182 and the inner peripheral surface portions of the tubular portions PC1a, PC2a (non-engagement state), and relative rotation between the second shaft 182 and the tubular portions PC1a, PC2a is allowed (forced free state, see FIG. 5).

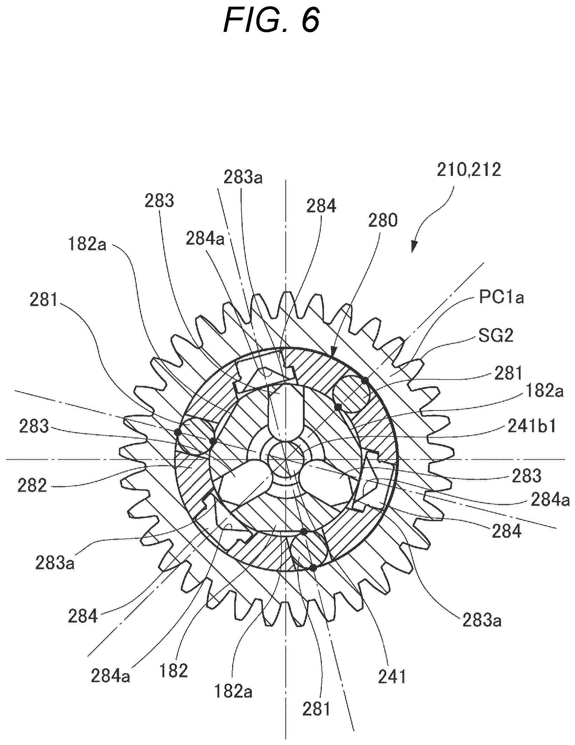

On the other hand, when the rollers 281 are allowed to move in the circumferential direction relative to the second shaft 182, the rollers 281 mesh with the outer peripheral surface portion of the second shaft 182 and the inner peripheral surface portions of the tubular portions PC1a, PC2a (engagement state), and the second shaft 182 and the tubular portions PC1a, PC2a are connected to be rotatable integrally in two directions (forced free release state, see FIG. 6).

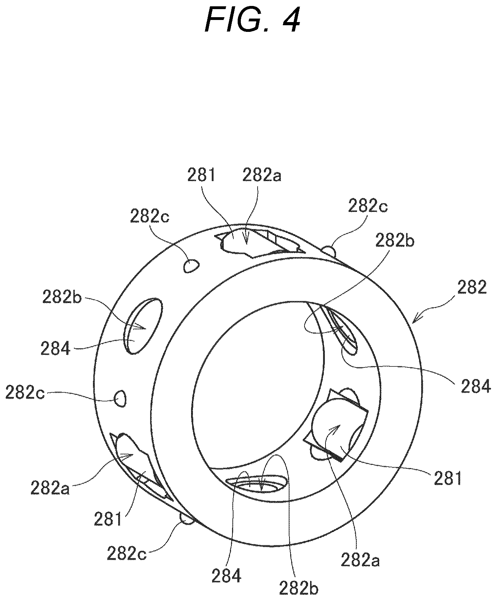

As shown in FIG. 4, the retainer 282 has a ring shape capable of relatively rotating relative to the second shaft 182 and the tubular portions PC1a, PC2a, and includes a plurality of roller holding portions 282a that hold the rollers 281 and a plurality of guide holding portions 282b that hold the guides 284.

In an outer peripheral surface of the retainer 282, a plurality of rubber balls 282c are embedded at predetermined intervals in the circumferential direction. These rubber balls 282c prevent unintended idling in the forced free release state by generating moderate friction between the tubular portions PC1a, PC2a and the retainer 282. Members for generating friction between the tubular portions PC1a, PC2a and the retainer 282 are not limited to the rubber balls 282c, and may be O-rings.

Returning to FIG. 5, each of the pins 283 includes a conical protrusion portion 283a on an outer end in the radial direction, and each of the guides 284 includes, on an inner end surface thereof in the radial direction, a conical recess portion 284a that fits (engages with) the protrusion portion 283a. When the protrusion portion 283a of the pin 283 is fitted to the recess portion 284a of the guide 284, a relative rotation position of the retainer 282 relative to the second shaft 182 is positioned at a predetermined position where the forced free state is obtained, due to a guiding effect of the pin 283 and the guide 284.

Returning to FIG. 1, the operation mechanism 240 includes an operation rod 241 provided to intermittently operate the connection and disconnection units 212, 222, and a servo motor 242 that linearly moves the operation rod 241. The second shaft 182 is a hollow shaft having an internal space S extending in the rotation axis direction (also referred to as the upper-lower direction), and the operation rod 241 is disposed in the internal space S.

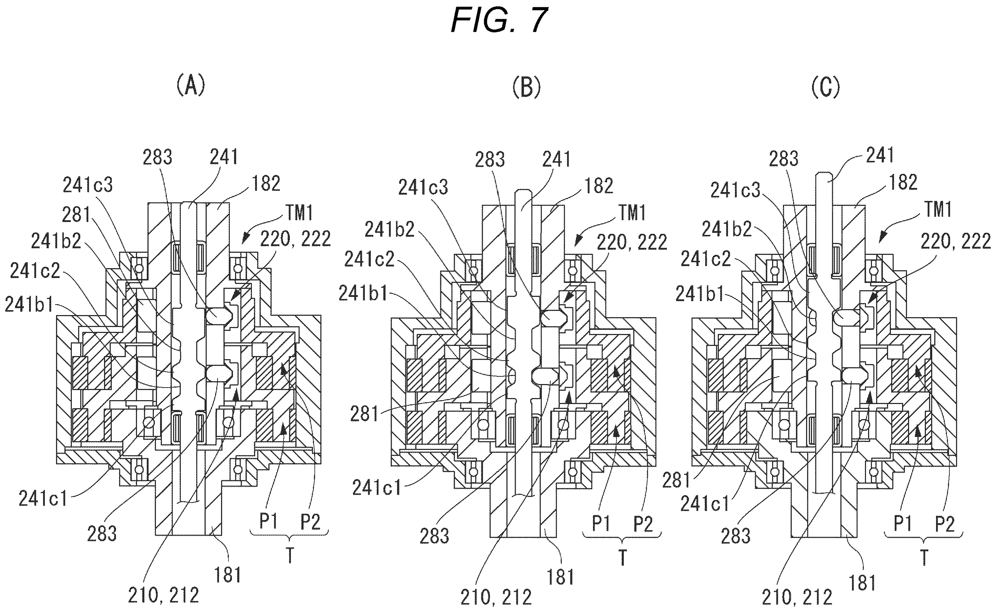

Referring to FIG. 7 again, small-diameter portions 241b1, 241b2 and large-diameter portions 241c1 to 241c3, which will be described later, are formed on an outer peripheral portion of the operation rod 241, and abut on inner-diameter end portions (inner ends) of the pins 283. When the small-diameter portions 241b1, 241b2 and the large-diameter portions 241c1 to 241c3 move the pins 283 forward and backward or enable the pins 283 to move forward and backward in the radial direction of the second shaft 182 in response to a position of the operation rod 241, the states of the connection and disconnection units 212, 222 are switched.

More specifically, as shown in FIG. 7, on the outer peripheral portion of the operation rod 241, in the order from below, the first large-diameter portion 241c1, the first small-diameter portion 241b1, the second large-diameter portion 241c2, the second small-diameter portion 241b2, and the third large-diameter portion 241c3 are formed with predetermined lengths and intervals therebetween. The operation rod 241 is provided to be capable of simultaneously controlling the two connection and disconnection units 212, 222, but may be provided separately for each of the connection and disconnection units 212, 222.

As shown in FIG. 7, the connection and disconnection units 212, 222 are switched between the forced free state (hereinafter referred to as an off state as appropriate) and the forced free release state (hereinafter referred to as an on state as appropriate) by the operation mechanism 240.

When the operation rod 241 of the operation mechanism 240 is at a lower position shown in (A) of FIG. 7, the third large-diameter portion 241c3 pushes the pin 283 of the connection and disconnection unit 222 outward in the radial direction while the second large-diameter portion 241c2 pushes the pin 283 of the connection and disconnection unit 212 outward in the radial direction, thus setting the connection and disconnection unit 222 and the connection and disconnection unit 212 to the off state. That is, the first power transmission path and the second power transmission path are disconnected. Thus, the power transmission device TM1 enters a neutral state where power transmission from the first shaft 181 to the second shaft 182 is cut off.

Further, when the operation rod 241 of the operation mechanism 240 is at a middle position shown in (B) of FIG. 7, the third large-diameter portion 241c3 pushes the pin 283 of the connection and disconnection unit 222 outward in the radial direction while the first small-diameter portion 241b1 allows the pin 283 of the connection and disconnection unit 212 to return inward in the radial direction, thereby setting the connection and disconnection unit 212 to the on state and the connection and disconnection unit 222 to the off state. That is, the first power transmission path is in the connection state, and the second power transmission path is in the disconnection state. Thus, the power transmission device TM1 enters a high-speed rotation state where the rotation power of the first shaft 181 is decelerated through the first power transmission path (first planetary mechanism P1) and transmitted to the second shaft 182.

Further, when the operation rod 241 of the operation mechanism 240 is at an upper position shown in (C) of FIG. 7, the second small-diameter portion 241b2 allows the pin 283 of the connection and disconnection unit 222 to return inward in the radial direction while the first large-diameter portion 241c1 pushes the pin 283 of the connection and disconnection unit 212 outward in the radial direction, thus setting the connection and disconnection unit 212 to the off state and the connection and disconnection unit 222 to the on state. That is, the first power transmission path is in the disconnection state, and the second power transmission path is in the connection state. Thus, the power transmission device TM1 is in a high torque state where the rotation power of the first shaft 181 is decelerated through the second power transmission path (the first planetary mechanism P1 and the second planetary mechanism P2) and transmitted to the second shaft 182.

In the two-way clutch 280 constituting the connection and disconnection units 212, 222, the roller 281 is an engagement element, and the operation rod 241, the pin 283, the guide 284, and the retainer 282 correspond to an operation unit that operates the engagement element (the roller 281) between the engagement state and the non-engagement state. Further, among the elements constituting the operation unit, the guide 284 and the retainer 282 correspond to an actuation element that moves the engagement element (the roller 281), and the operation rod 241 and the pin 283 correspond to an operation element provided to be able to operate the actuation element. The operation element may be capable of operating the engagement element (roller 281) not via the actuation element. Further, among the elements constituting the actuation element, the pin 283 is an advance and retraction element provided to be capable of advancing and retracting along the radial direction, and the operation rod 241 corresponds to an extending portion that allows advancing and retracting of the advance and retraction element (the pin 283).

In this way, since the transmission T of the power transmission device TM1 includes the first planetary mechanism P1 and the second planetary mechanism P2, the transmission ratio can be increased in a small space. In particular, since the second power transmission path passes through the first planetary mechanism P1 in addition to the second planetary mechanism P2, the transmission ratio can be effectively increased.

Since the rotation axes of the carriers PC1, PC2 of the first planetary mechanism P1 and the second planetary mechanism P2 coincide with the rotation axes of the first shaft 181 and the second shaft 182, the transmission T can be disposed coaxially with the first shaft 181 and the second shaft 182, and the power transmission device TM1 can be reduced in size.

The connection and disconnection mechanism 20 includes the operation rod 241 disposed in the internal space S of the second shaft 182 along the rotation axis direction, and disconnects and connects the first power transmission path and the second power transmission path based on a movement control of the operation rod 241. Thus, the connection and disconnection mechanism 20 can be concentrated inside the transmission T, and the power transmission device TM1 can be further reduced in size.

In the power transmission device TM1 of the present embodiment, the first connection and disconnection mechanism 210 is disposed at a downstream side with respect to the first planetary mechanism P1 in the first power transmission path, and the second connection and disconnection mechanism 220 is disposed at a downstream side with respect to the first planetary mechanism P1 and the second planetary mechanism P2 in the second power transmission path. Thus, when the power is input from the downstream side (second shaft 182), the number of accompanying members can be reduced.

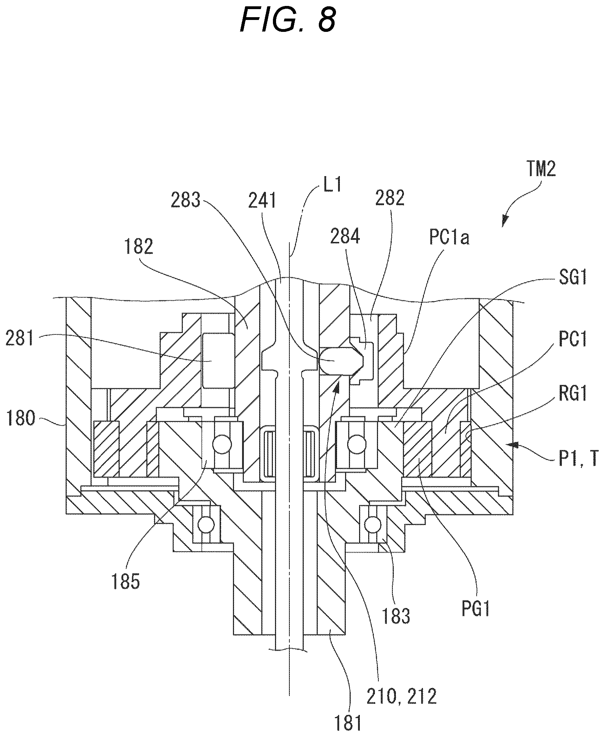

In the following description, other embodiments of the present invention will be described with reference to FIG. 8 and subsequent drawings. Here, the same reference numerals as in the first embodiment are used for the same configurations as in the first embodiment, and the description of the first embodiment may be incorporated.

### (Second Embodiment)

As described above, the power transmission device TM1 of the first embodiment includes the two planetary mechanisms P1, P2 as the transmission T, includes the two connection and disconnection mechanisms 210, 220 as the connection and disconnection mechanism 20, and switches to the neutral state, the high-speed rotation state, and the high torque state based on the control of the connection and disconnection mechanisms 210, 220. On the other hand, as shown in FIG. 8, a power transmission device TM2 of a second embodiment is different from the power transmission device TM2 of the first embodiment in including one planetary mechanism P1 as the transmission T, one connection and disconnection mechanism 210 as the connection and disconnection mechanism 20, and switching between the neutral state and the high-speed rotation state based on the control of the connection and disconnection mechanism 210. According to the second embodiment, the power transmission device TM2 can be made smaller and simpler.

### (Third Embodiment)

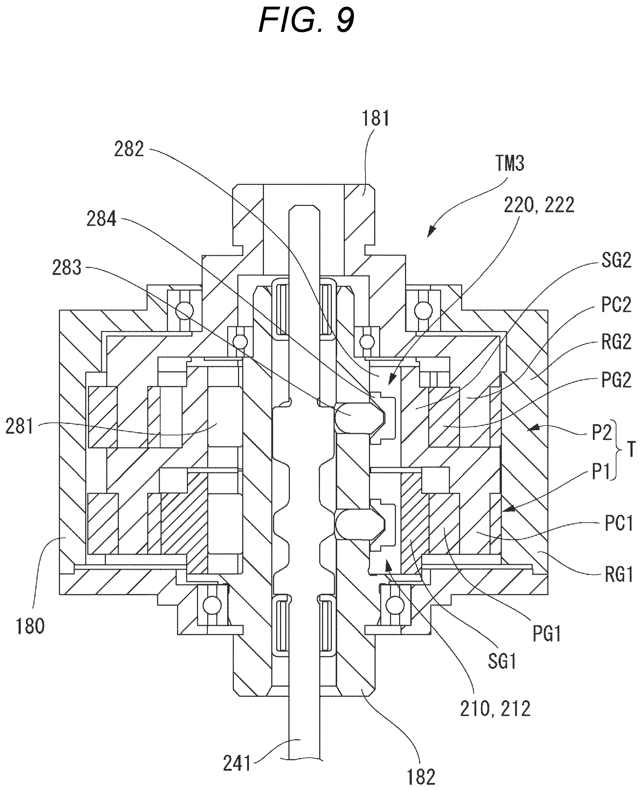

As shown in FIG. 9, a power transmission device TM3 of a third embodiment is different from the power transmission device TM1 of the first embodiment in that the second shaft 182 is set as an input shaft and the first shaft 181 is set as an output shaft. Specifically, in the power transmission device TM3 of the third embodiment, the sun gear SG1 of the first planetary mechanism P1 is connected to the second shaft 182 via the first connection and disconnection mechanism 210. The carrier PC1 of the first planetary mechanism P1 is formed integrally with the sun gear SG2 of the second planetary mechanism P2. The sun gear SG2 of the second planetary mechanism P2 is connected to the second shaft 182 via the second connection and disconnection mechanism 220, and the carrier PC2 is formed integrally with the first shaft 181.

That is, the power transmission device TM3 of the third embodiment enters the neutral state where the power transmission from the second shaft 182 to the first shaft 181 is cut off, when the first connection and disconnection mechanism 210 and the second connection and disconnection mechanism 220 are set to the off state. Further, when the first connection and disconnection mechanism 210 is set to the on state and the second connection and disconnection mechanism 220 is set to the off state, the high torque state is obtained in which the rotation power of the second shaft 182 is transmitted to the first shaft 181 via the first planetary mechanism P1 and the second planetary mechanism P2. Further, when the first connection and disconnection mechanism 210 is set to the off state and the second connection and disconnection mechanism 220 is set to the on state, the high-speed rotation state is obtained in which the rotation power of the second shaft 182 is transmitted to the first shaft 181 via the second planetary mechanism P2. According to the third embodiment, the neutral state, the high torque state, and the high-speed rotation state can be switched with an arrangement configuration different from that of the first embodiment.

### (Fourth Embodiment)

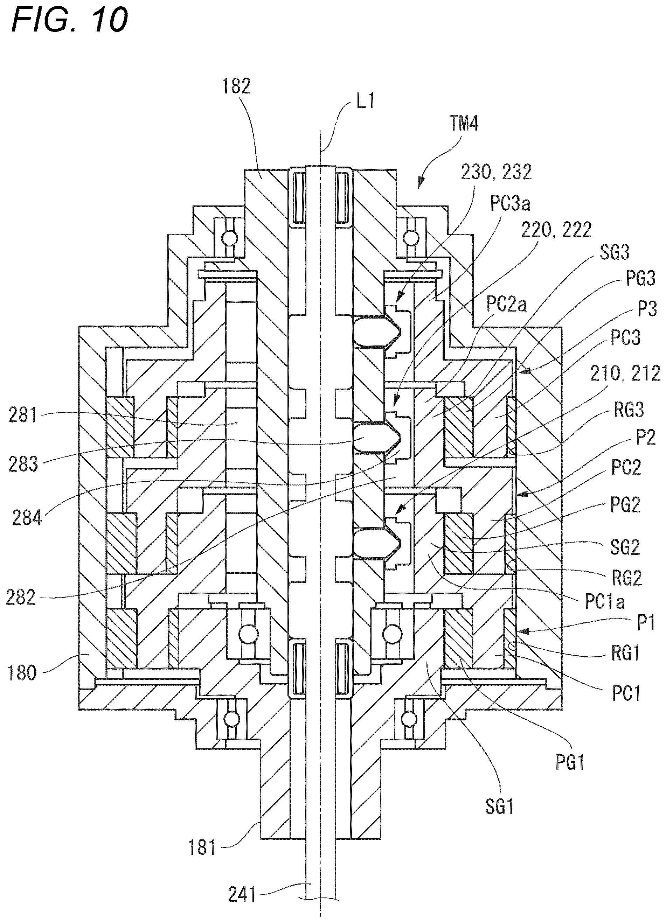

As shown in FIG. 10, a power transmission device TM4 of a fourth embodiment is different from the power transmission device TM1 of the first embodiment in including three planetary mechanisms P1, P2, and P3 as the transmission T and three connection and disconnection mechanisms 210, 220, and 230 as the connection and disconnection mechanism 20, and including the neutral state and three-stage speed change states (a first-speed state (high torque state), a second-speed state (intermediate rotation/intermediate torque state), and a third-speed state (high-speed rotation state)).

Specifically, in addition to the power transmission device TM1 of the first embodiment, the third planetary mechanism P3 is disposed above the second planetary mechanism P2. The third planetary mechanism P3 includes a sun gear SG3, a ring gear RG3, a plurality of planetary gears PG3 that mesh with the sun gear SG3 and the ring gear RG3, and a carrier PC3 that supports the planetary gears PG3 in a rotatable and revolvable manner, and the three rotation elements including the sun gear SG3, the ring gear RG3, and the carrier PC3 satisfy a collinear relationship in which respective rotation speeds are always aligned on a single straight line in a velocity collinear diagram (also referred to as a collinear diagram).

The carrier PC2 of the second planetary mechanism P2 is formed integrally with the sun gear SG3 of the third planetary mechanism P3. In the present embodiment, the sun gear SG3 is formed on an outer peripheral portion of a tubular portion PC2a integrally formed with the carrier PC2. Thus, the rotation power decelerated by the second planetary mechanism P2 can be transmitted to the third planetary mechanism P3.

The sun gear SG3 (tubular portion PC2a) of the third planetary mechanism P3 is connected to the second shaft 182 via the second connection and disconnection mechanism 220. Thus, the second power transmission path including the first planetary mechanism P1 and the second planetary mechanism P2 is formed, which is similar to the TM1 of the first embodiment.

The ring gear RG3 of the third planetary mechanism P3 is formed integrally with the case 180. That is, the ring gear RG3 is not rotatable. The ring gear RG3 of the third planetary mechanism P3 does not necessarily have to be formed integrally with the case 180, and may be formed separately from the case 180 and fixed to the case 180 in a non-rotatable manner.

Thus, the carrier PC3 of the third planetary mechanism P3 decelerates and outputs the rotation received from the sun gear SG3.

The carrier PC3 of the third planetary mechanism P3 is connected to the second shaft 182 via the third connection and disconnection mechanism 230. In the present embodiment, the third connection and disconnection mechanism 230 is provided between the tubular portion PC3a integrally formed with the carrier PC3 and the second shaft 182 located on an inner peripheral portion of the tubular portion PC3a. Although not shown, the bearings that allow relative rotation may be provided between the rotation elements of the first planetary mechanism P1 and the rotation elements of the second planetary mechanism P2, between the rotation elements of the second planetary mechanism P2 and the rotation elements of the third planetary mechanism P3, and between the case 180 and the rotation elements of the first planetary mechanism P1, the second planetary mechanism P2, or the third planetary mechanism P3.

In the transmission T configured in this way, the first power transmission path for transmitting the power of the power source received from the first shaft 181 to the second shaft 182 via the first planetary mechanism P1 and the first connection and disconnection mechanism 210 is formed, the second power transmission path for transmitting the power to the second shaft 182 via the first planetary mechanism P1, the second planetary mechanism P2, and the second connection and disconnection mechanism 220 is formed, and a third power transmission path for transmitting the power to the second shaft 182 via the first planetary mechanism P1, the second planetary mechanism P2, the third planetary mechanism P3, and the third connection and disconnection mechanism 230 is formed.

The third connection and disconnection mechanism 230 includes a connection and disconnection unit 232 provided between the tubular portion PC3a and the second shaft 182. A configuration of the connection and disconnection unit 232 is the same as those of the connection and disconnection units 212, 222.

In the power transmission device TM4 of the fourth embodiment, when the connection and disconnection unit 212, the connection and disconnection unit 222, and the connection and disconnection unit 232 are set to the off state, the first to third power transmission paths are in the disconnection state, and the neutral state is obtained in which the power transmission from the first shaft 181 to the second shaft 182 is cut off.

When the connection and disconnection unit 212 is set to the on state and the connection and disconnection unit 222 and the connection and disconnection unit 232 are set to the off state, the first power transmission path is in the connection state, the second power transmission path and the third power transmission path are in the disconnection state, and the third-speed state (high-speed rotation state) is obtained in which the rotation power of the first shaft 181 is decelerated through the first power transmission path (first planetary mechanism P1) and transmitted to the second shaft 182.

When the connection and disconnection unit 222 is set to the on state and the connection and disconnection unit 212 and the connection and disconnection unit 232 are set to the off state, the second power transmission path is in the connection state, the first power transmission path and the third power transmission path are in the disconnection state, and the second-speed state (intermediate rotation/intermediate torque state) is obtained in which the rotation power of the first shaft 181 is decelerated through the second power transmission path (the first planetary mechanism P1 and the second planetary mechanism P2) and transmitted to the second shaft 182.

When the connection and disconnection unit 232 is set to the on state and the connection and disconnection unit 212 and the connection and disconnection unit 222 are set to the off state, the third power transmission path is in the connection state, the first power transmission path and the second power transmission path are in the disconnection state, and the first-speed state (high torque state) is obtained in which the rotation power of the first shaft 181 is decelerated through the third power transmission path (the first planetary mechanism P1, the second planetary mechanism P2, and the third planetary mechanism P3) and transmitted to the second shaft 182. As described above, according to the power transmission device TM4 of the fourth embodiment, one speed-change stage can be added to the power transmission device TM1 of the first embodiment by increasing one planetary mechanism (third planetary mechanism P3) and one connection and disconnection mechanism (third connection and disconnection mechanism 230). Accordingly, the number of speed-change stages of the power transmission device TM4 may be four or more.

### (Modification of Fourth Embodiment)

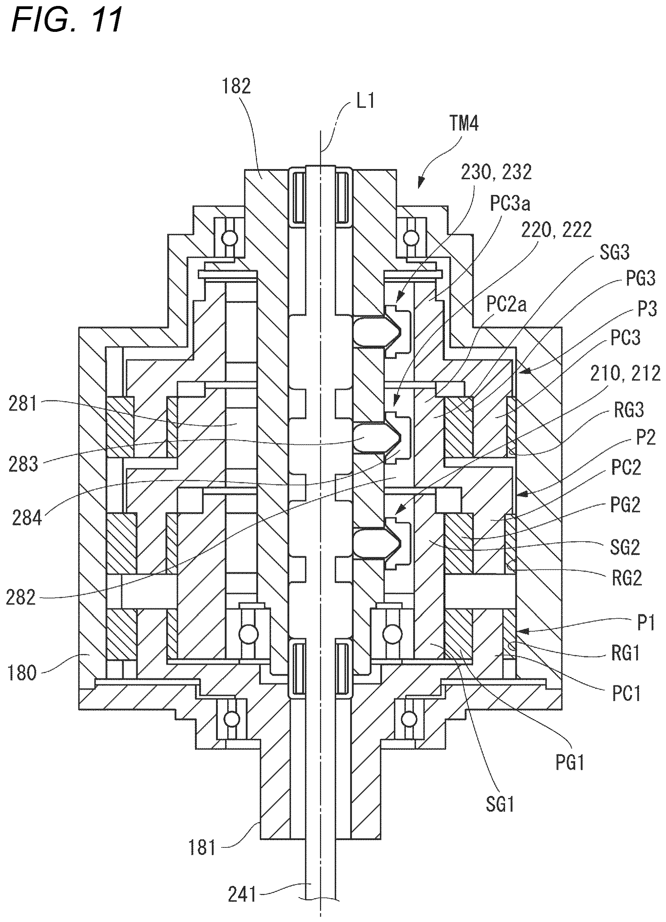

As shown in FIG. 11, the power transmission device TM4 according to a modification of the fourth embodiment is the same as the power transmission device TM4 of the fourth embodiment in including three planetary mechanisms P1, P2, and P3 as the transmission T and three connection and disconnection mechanisms 210, 220, and 230 as the connection and disconnection mechanism 20, and including the neutral state and the three-stage speed change states (the first-speed state (high torque state), the second-speed state (intermediate rotation/intermediate torque state), and the third-speed state (high-speed rotation state)).

In the transmission T of the present modification, the carrier PC1 of the first planetary mechanism P1 is formed integrally with the first shaft 181. The carrier PC1 of the first planetary mechanism P1 does not necessarily have to be formed integrally with the first shaft 181, and may be formed separately from the first shaft 181 and connected to the first shaft 181 in an integrally rotatable manner. Thus, the rotation power of the first shaft 181 is input to the first planetary mechanism P1. Further, the sun gear SG1 of the first planetary mechanism P1 is formed integrally with the sun gear SG2 of the second planetary mechanism P2. The sun gear SG1 of the first planetary mechanism P1 does not necessarily have to be formed integrally with the sun gear SG2 of the second planetary mechanism P2, and may be formed separately from the sun gear SG2 of the second planetary mechanism P2 and connected to the sun gear SG2 of the second planetary mechanism P2 in an integrally rotatable manner. Thus, the rotation power accelerated by the first planetary mechanism P1 can be transmitted to the second planetary mechanism P2. Although not shown, the bearings that allow relative rotation may be provided between the rotation elements of the first planetary mechanism P1 and the rotation elements of the second planetary mechanism P2, between the rotation elements of the second planetary mechanism P2 and the rotation elements of the third planetary mechanism P3, and between the case 180 and the rotation elements of the first planetary mechanism P1, the second planetary mechanism P2, or the third planetary mechanism P3.

In the transmission T configured in this way, the first power transmission path for transmitting the power of the power source received from the first shaft 181 to the second shaft 182 via the first planetary mechanism P1 and the first connection and disconnection mechanism 210 is formed, the second power transmission path for transmitting the power to the second shaft 182 via the first planetary mechanism P1, the second planetary mechanism P2, and the second connection and disconnection mechanism 220 is formed, and the third power transmission path for transmitting the power to the second shaft 182 via the first planetary mechanism P1, the second planetary mechanism P2, the third planetary mechanism P3, and the third connection and disconnection mechanism 230 is formed.

The transmission T of the present modification can be set such that the rotation of the first shaft 181 is transmitted to the second shaft 182 at an increased speed through the first power transmission path, is transmitted to the second shaft 182 at substantially the same speed through the second power transmission path, and is transmitted to the second shaft 182 at a decreased speed through the third power transmission path.

### (Fifth Embodiment)

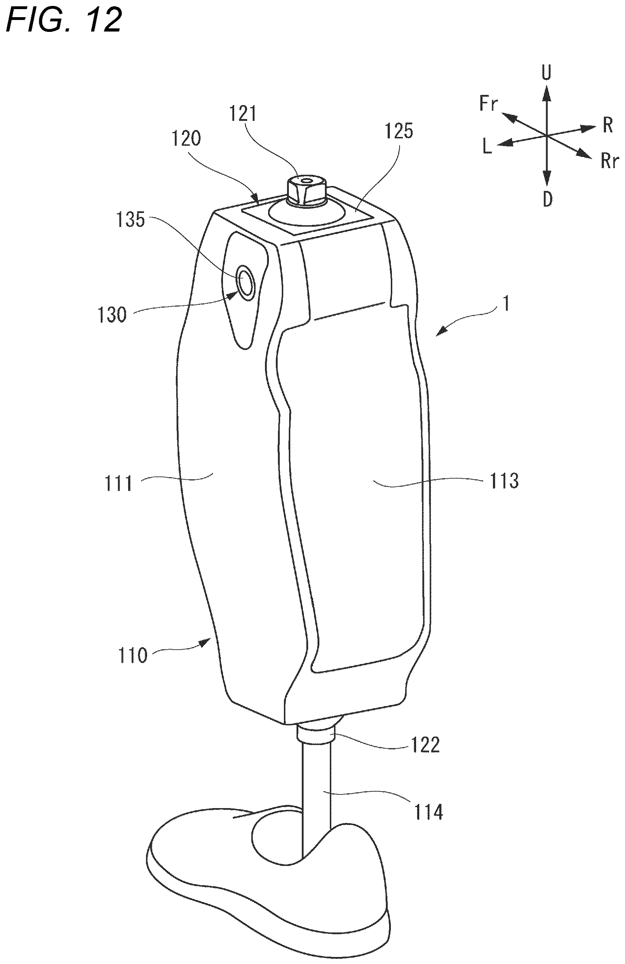

In the following description, an electric prosthetic leg 1 (fifth embodiment) in which the power transmission device TM1 of the first embodiment is assembled will be described with reference to FIGS. 12 to 16. In the following description, a front-rear direction, a left-right direction, and an upper-lower direction are defined with reference to a user of the electric prosthetic leg 1. In the drawings, a front side of the electric prosthetic leg is denoted by Fr, a rear side is denoted by Rr, a left side is denoted by L, a right side is denoted by R, an upper side is denoted by U, and a lower side is denoted by D.

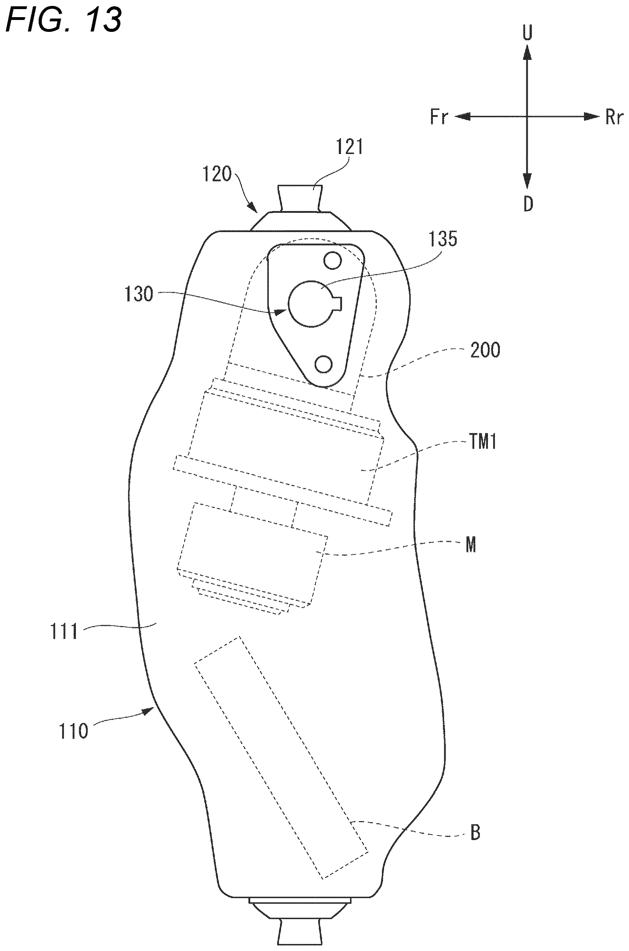

As shown in FIGS. 12 and 13, the electric prosthetic leg 1 is a prosthetic leg that is attached to a leg of a person who does not have a knee. The electric prosthetic leg 1 includes: a below-knee member 110 positioned on a lower side of the knee, an above-knee member 120 positioned on an upper side of the knee and attached to a thigh, a knee joint mechanism 130 that couples the below-knee member 110 and the above-knee member 120 such that a formed angle formed therebetween is variable, an enlarging and reducing device 200 capable of enlarging and reducing the formed angle formed between the below-knee member 110 and the above-knee member 120, and a battery B that supplies power to the enlarging and reducing device 200 and the like.

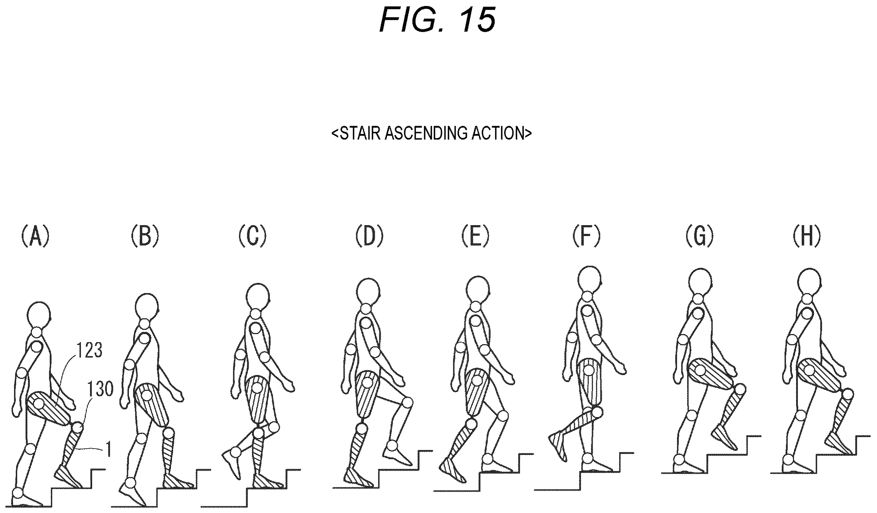

The above-knee member 120 includes an adapter 121 to be coupled to a socket which is not shown, an upper wall portion 125 to which the adapter 121 is attached, and a pair of left and right side wall portions (not shown) coupled to upper wall portion 125. The socket is a joint member to be provided on a thigh 123 (see FIGS. 15 and 15), and the above-knee member 120 is integrated with the thigh 123 by coupling the adapter 121 to the socket.

The below-knee member 110 includes a box-shaped main frame 111 having an open rear portion, a detachable rear cover 113 that covers the rear opening of the main frame 111 in an openable and closable manner, and an adapter 122 attached to a lower surface of the main frame 111.

The above-knee member 120 is provided on an upper portion of the main frame 111 of the below-knee member 110 via a coupling shaft 135 that constitutes the knee joint mechanism 130, and a leg 114 extending downward is coupled to the adapter 122 of the main frame 111.

The enlarging and reducing device 200 capable of enlarging and reducing the formed angle formed between the below-knee member 110 and the above-knee member 120 is provided in a space formed between the below-knee member 120 and the above-knee member 110. The enlarging and reducing device 200 is configured to enlarge and reduce the formed angle formed between the below-knee member 110 and the above-knee member 120 by meshing of gears.

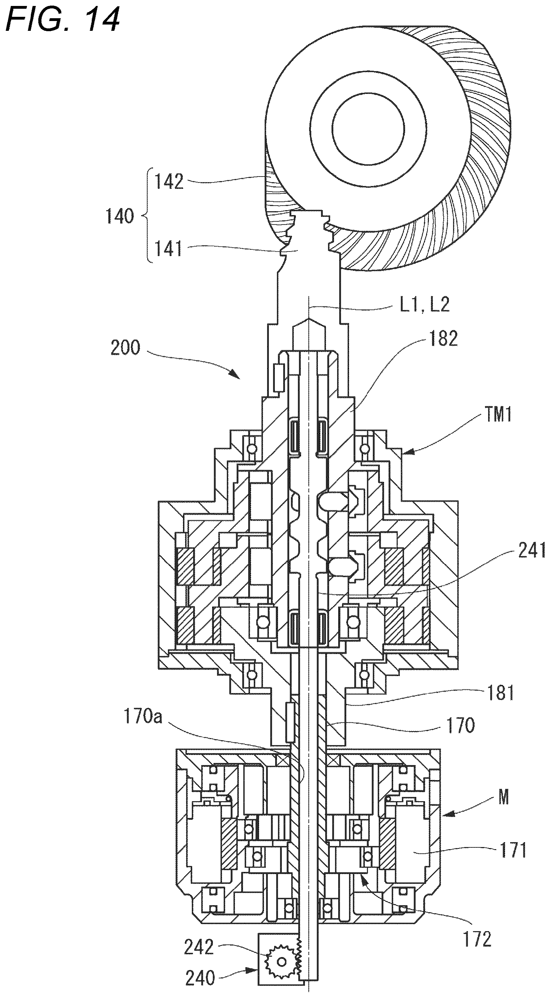

As shown in FIGS. 13 and 14, the enlarging and reducing device 200 includes a motor M that outputs rotation power, the power transmission device TM1 that transmits the power of the motor M, and a bevel gear mechanism 140 that can enlarge and reduce the formed angle formed between the below-knee member 110 and the above-knee member 120.

The motor M is, for example, a permanent magnet motor and is disposed below the power transmission device TM1. The motor M is a motor with a built-in gear mechanism, including a motor body 171, a gear mechanism unit 172 that decelerates output rotation of the motor body 171, and an output shaft 170 that outputs the accelerated rotation power. The output shaft 170 is a hollow cylindrical shaft having an internal space 170a along the rotation axis direction.

The bevel gear mechanism 140 includes a first bevel gear 141 that is disposed on a side opposite to the motor M with respect to the power transmission device TM1 on a power transmission path of the motor M and is supported by the below-knee member 110, and a second bevel gear 142 that meshes with the first bevel gear 141 in a manner of being capable of transmitting rotation and is supported by the above-knee member 120.

The first bevel gear 141 is configured to integrally rotate with the second shaft 182 which is an output element of the power transmission device TM1. The first bevel gear 141 transmits, to the second bevel gear 142, the power of the motor M transmitted via the power transmission device TM1.

The second bevel gear 142 is provided to be rotatable relative to the coupling shaft 135 and rotatable integrally with the above-knee member 120. Thus, when the second bevel gear 142 meshing with the first bevel gear 141 rotates, the above-knee member 120 rotates about the coupling shaft 135. Therefore, the formed angle formed between the above-knee member 120 and the below-knee member 110 varies.

As shown in FIG. 14, the power transmission device TM1 is disposed such that the rotation axis L1 of the first shaft 181 and the second shaft 182 corresponds to a rotation axis L2 of the output shaft 170 of the motor M and the first bevel gear 141. The first shaft 181 is connected to the output shaft 170 of the motor M in an integrally rotatable manner, and inputs the rotation power of the motor M to the power transmission device TM1. The second shaft 182 is connected to the first bevel gear 141 in an integrally rotatable manner, and transmits, to the first bevel gear 141, the rotation power of the motor M which is speed-changed by the power transmission device TM1. Further, the operation rod 241 extends below the motor M via the internal space 170a formed in the output shaft 170 of the motor M, and is controlled to move by a servo motor 242 provided below the motor M. Since the power transmission device TM1 has two power transmission paths having different transmission ratios, an action speed and generated power for stretching and bending in the knee joint mechanism 130 can be switched.

In the electric prosthetic leg 1 configured in this way, it is possible to smoothly ascend stairs, which has been required to be done one by one by a leg on a non-prosthetic leg side, with a passive prosthetic leg including a passive damper in the related art. FIG. 15 is a diagram showing actions (stair ascending actions) of the user and the electric prosthetic leg 1 when ascending stairs. (A) to (D) of FIG. 15 are diagrams showing a stance phase, (D) and (E) of FIG. 15 are diagrams showing a transition phase from a stance to an initial swing, (E) to (G) of FIG. 15 are diagrams showing an initial swing phase, (G) of FIG. 15 is a diagram showing a transition phase from the initial swing to a terminal swing and a terminal swing phase, and (H) of FIG. 15 is a diagram showing a transition phase from the terminal swing to the stance and the stance phase.

Specifically, as shown in (A) to (D) of FIG. 15, large power is required when the knee joint mechanism 130 is stretched from a bending state, in a state where a load is applied to the electric prosthetic leg 1 when moving the electric prosthetic leg 1 forward to ascend stairs.

At this time, the power transmission device TM1 is set to the high torque state shown in (C) of FIG. 7. In this state, when the motor M is rotated in a normal rotation direction, the second bevel gear 142 fixed to the above-knee member 120 rotates as the first bevel gear 141 fixed to the below-knee member 110 rotates, and thereby the above-knee member 120 rotates about the coupling shaft 135 with respect to the below-knee member 110, and knee joint mechanism 130 is stretched. Then, since the power for the stretching is power whose torque is increased, even when a large load is applied to the electric prosthetic leg 1 when moving the electric prosthetic leg 1 forward to ascend stairs, the knee joint mechanism 130 can be reliably stretched from the bending state.

On the other hand, in order to smoothly ascend stairs, as shown in (E) to (H) of FIG. 15, it is necessary to bend (raise) the knee joint mechanism 130 from the stretched state while a load is applied to a healthy leg. When the knee joint mechanism 130 is bent from the stretched state, large power is not required, but quick action is required.

At this time, the power transmission device TM1 is set to the high-speed rotation state shown in (B) of FIG. 7. In this state, when the motor M is rotated in a reverse rotation direction, the second bevel gear 142 fixed to the above-knee member 120 rotates as the first bevel gear 141 fixed to the below-knee member 110 rotates, and thereby the above-knee member 120 rotates about the coupling shaft 135 with respect to the below-knee member 110, and knee joint mechanism 130 is bent. Since the power for the bending is power whose rotation speed is high, the knee joint mechanism 130 can be bent quickly.

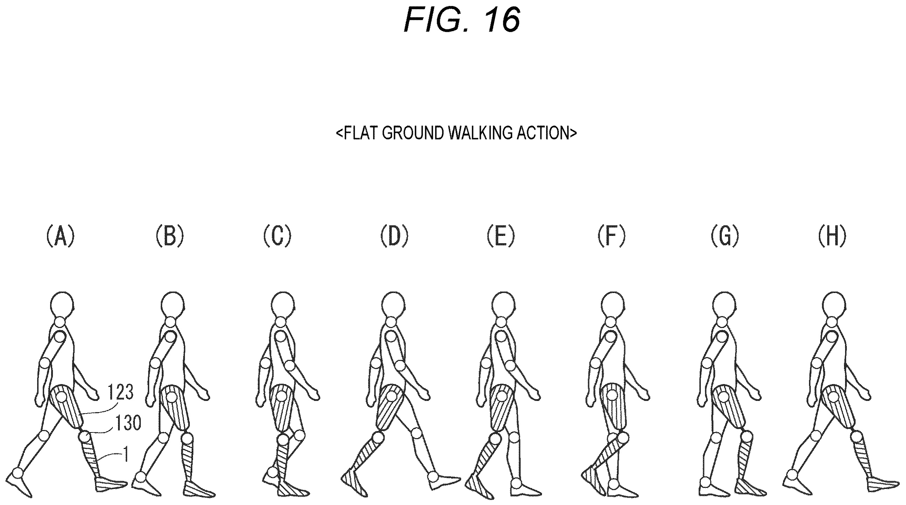

FIG. 16 is a diagram showing actions (flat ground walking actions) of the user and the electric prosthetic leg when walking on a flat ground. (A) to (D) of FIG. 16 are diagrams showing the stance phase, (D) of FIG. 16 is a diagram showing a transition phase from the stance to a swing, (E) to (H) of FIG. 16 are diagrams showing a swing phase, and (H) of FIG. 16 is a diagram showing a transition phase from the swing to the stance, and the stance phase.

During walking on the flat ground shown in FIG. 16 and during descending of stairs (stair descending), as shown in (A) to (D) of FIG. 16, in a state where a load is applied to the electric prosthetic leg 1, the power transmission device TM1 is set to the high-speed rotation state shown in (C) of FIG. 7. In this state, when the motor M is in a non-driven state, an external force in a bending direction acting on the electric prosthetic leg 1 is transmitted from the bevel gear mechanism 140 to the motor M via the first planetary mechanism P1 of the power transmission device TM1, so that friction between the motor M and the first planetary mechanism P1 is utilized to dampen the external force in the bending direction, thus preventing a so-called giving way.

As shown in (D) to (H) of FIG. 16, in a state where a load is applied to the healthy leg, the power transmission device TM1 is set to the neutral state shown in (A) of FIG. 7. In this state, since the motor M and the bevel gear mechanism 140 are in a power non-transmittable state, the user of the electric prosthetic leg 1 can smoothly swing the leg.

### (Sixth Embodiment)

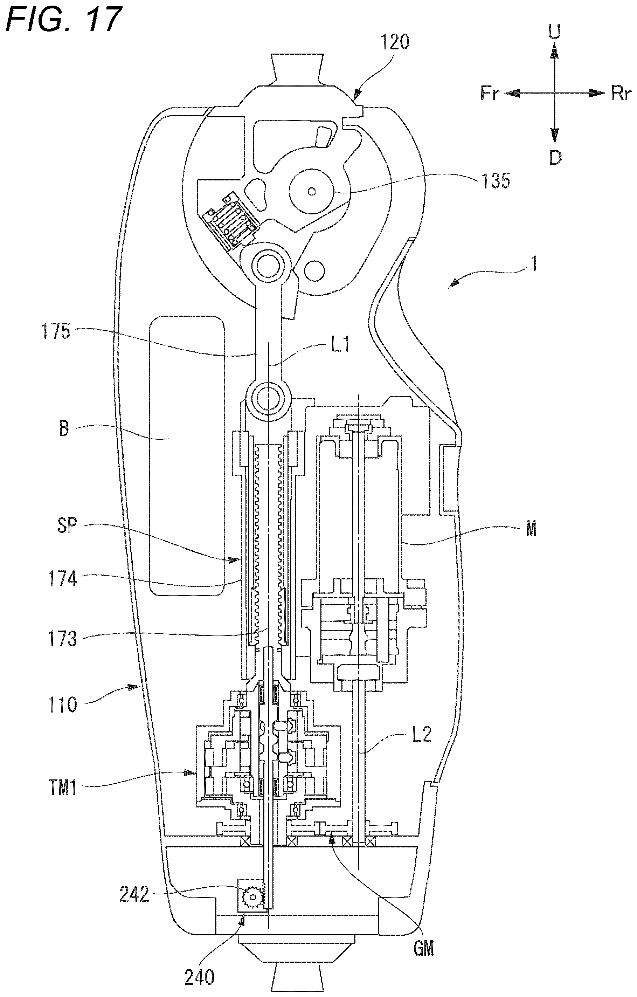

The electric prosthetic leg 1 shown in FIG. 17 is a modification of the fifth embodiment, and is different from the fifth embodiment in that the electric prosthetic leg 1 is bent and stretched using a spindle unit SP instead of the bevel gear mechanism 140. The spindle unit SP includes a spindle 173 formed with a male screw and a sleeve 174 formed with a female screw, and rotation of the spindle 173 causes the sleeve 174 to perform translational motion along a rotation axis of the spindle 173. Specifically, the spindle 173 rotates after receiving the rotation power of the motor M transmitted by the power transmission device TM1. On the other hand, the sleeve 174 is non-rotatably and movably in the upper-lower direction supported by the below-knee member 110. Therefore, when the spindle 173 rotates to one side after receiving the rotation power of the motor M transmitted by the power transmission device TM1, the sleeve 174 is translated away from the power transmission device TM1, and when the spindle 173 rotates to the other side, the sleeve 174 is translated in a direction of approaching the power transmission device TM1. The translation operation of the sleeve 174 away from the power transmission device TM1 may be referred to as an expanding operation of the spindle unit SP, and conversely, the translation operation of the sleeve 174 approaching the power transmission device TM1 may be referred to as a contracting operation of the spindle unit SP.

That is, a distance between the sleeve 174 and the power transmission device TM1 increases or decreases depending on a rotation direction of the spindle 173. An upper end of the sleeve 174 is coupled to the above-knee member 120 via a link member 175. As the distance between the sleeve 174 and the power transmission device TM1 increases or decreases depending on the rotation direction of the spindle 173, the below-knee member 110 and the above-knee member 120 rotate around the coupling shaft 135. Thus, the formed angle formed between the above-knee member 120 and the below-knee member 110 varies. When the formed angle formed between the above-knee member 120 and the below-knee member 110 is an acute angle between an acute angle and an obtuse angle, the knee joint mechanism 130 stretches as the formed angle increases, and the knee joint mechanism 130 bends as the angle decreases.

According to the sixth embodiment, operations and effects similar to those of the fifth embodiment are obtained. In the electric prosthetic leg 1 of the sixth embodiment, the spindle unit SP and the power transmission device TM1 are disposed such that respective rotation axes (rotation axis L1) coincide with each other. On the other hand, the motor M is disposed such that the rotation axis L2 is parallel to the rotation axis L1 of the spindle unit SP or the power transmission device TM1, and inputs the rotation power to the power transmission device TM1 via a spur gear mechanism GM.

### (Seventh Embodiment)

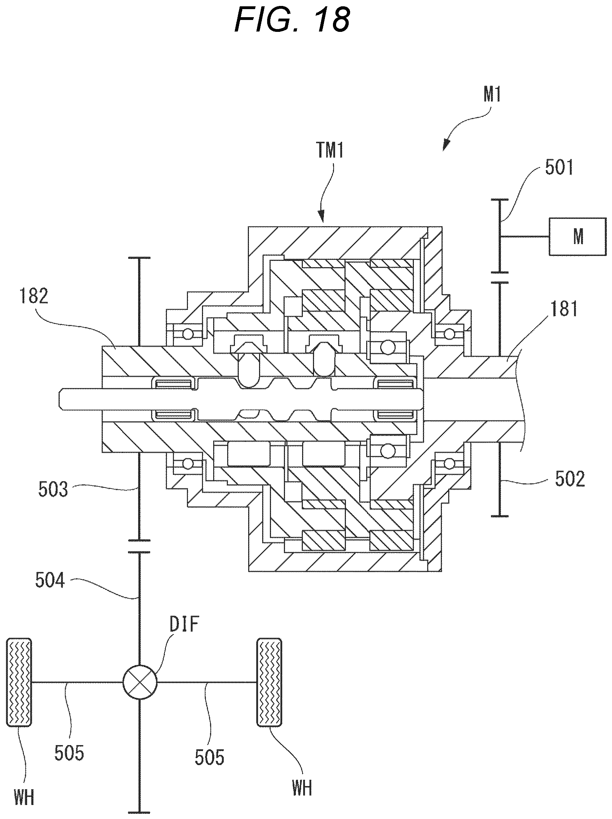

FIG. 18 shows a configuration of a moving object M1 in which the power transmission device TM1 of the first embodiment is assembled. The moving object M1 includes the motor M, a first gear 501 that outputs the rotation power of the motor M, a second gear 502 that is connected to the first shaft 181 of the power transmission device TM1 in an integrally rotatable manner and meshes with the first gear 501, a third gear 503 that is connected to the second shaft 182 of the power transmission device TM1 in an integrally rotatable manner and outputs the rotation power of the motor M speed-changed by the power transmission device TM1, a fourth gear 504 that meshes with the third gear 503 and transmits the rotation power to a differential device DIF, left and right axles 505 to which the rotation power is transmitted from the differential device DIF in a differentially rotatable manner, and left and right wheels WH that are provided at tip end portions of the axles 505 in an integrally rotatable manner.

According to the moving object M1 of the seventh embodiment, the power transmission device TM1 can be speed-changed to the neutral state, the high-speed rotation state, and the high torque state.

Although the various embodiments have been described above with reference to the drawings, it is needless to say that the present invention is not limited to these examples. It is apparent to those skilled in the art that various changes or modifications can be conceived within the scope described in the claims, and it is understood that the changes or modifications naturally fall within the technical scope of the present invention. In addition, respective constituent elements in the above-described embodiments may be freely combined without departing from the gist of the invention.

In the present specification, at least the following matters are described. Although corresponding constituent elements or the like in the embodiment described above are shown in parentheses, the present invention is not limited thereto.
(1) A power transmission device (power transmission devices TM1 to TM4) including:
   a connection and disconnection mechanism (connection and disconnection mechanism 20); and
   a transmission (transmission T), in which
   the connection and disconnection mechanism (connection and disconnection mechanism 20, first connection and disconnection mechanism 210) includes:
      an engagement element (roller 281) disposed between a first rotation member (tubular portion PC1a) and a second rotation member (second shaft 182); and
      an operation unit (operation rod 241, pin 283, retainer 282, and guide 284) configured to operate the engagement element to an engagement state where the first rotation member and the second rotation member are integrally rotatable and a non-engagement state where the first rotation member and the second rotation member are relatively rotatable,
   the operation unit includes an actuation element (retainer 282, guide 284) configured to move the engagement element, and an operation element (operation rod 241, pin 283) provided to be capable of operating the engagement element via the actuation element or capable of operating the engagement element not via the actuation element,
   the first rotation member and the second rotation member are disposed such that respective rotation axes (rotation axis L1) of the first rotation member and the second rotation member coincide with each other, and to at least a part of the first rotation member and the second rotation member overlap each other as viewed in a radial direction with respect to the rotation axes,
   the operation element includes:
      an advance and retraction element (pin 283) provided to be capable of advancing and retracting along the radial direction; and
      an extending portion (operation rod 241) extending along the rotation axes and provided to be capable of advancing and retracting along the rotation axes,
   the transmission includes a transmission unit (second planetary mechanism P2) including:
      a first rotation element (sun gear SG2) configured by the first rotation member or a rotation member rotating integrally with the first rotation member;
      a second rotation element (carrier PC2) provided to be capable of transmitting rotation with the first rotation element; and
      a third rotation element (ring gear RG2) provided to be capable of transmitting rotation with the second rotation element,
   the second rotation element and the third rotation element are:
      disposed outward than the second rotation member in the radial direction; and
   disposed such that at least a part of the second rotation element and the third rotation element are disposed overlaps the second rotation member as viewed in the radial direction.
   According to (1), the transmission can be concentrated around the second rotation member, and the power transmission device can be reduced in size.
(2) The power transmission device according to (1), in which
   the advance and retraction element is provided such that an inner end, which is an end portion of the advance and retraction element on a side of the rotation axes in the radial direction, abuts on the extending portion.
   According to (2), the inner end of the advance and retraction element abuts on the extending portion of the operation element, and thus the advance and retraction element can advance and retract along an orthogonal direction orthogonal to the rotation axes of the first rotation member and the second rotation member.
(3) The power transmission device according to (1) or (2), further including:
   a third rotation member (tubular portion PC2a) and a fourth rotation member (second shaft 182) disposed such that the rotation axes and rotation axes of the third rotation member and the fourth rotation member coincide with each other, in which
   the connection and disconnection mechanism (connection and disconnection mechanism 20, second connection and disconnection mechanism 220) further includes:
      another engagement element (roller 281) disposed between the third rotation member and the fourth rotation member; and
      another operation unit (operation rod 241, pin 283, retainer 282, guide 284) configured to operate the another engagement element to an engagement state where the third rotation member and the fourth rotation member are integrally rotatable and a non-engagement state where the third rotation member and the fourth rotation member are relatively rotatable.
   According to (3), by controlling the another engagement element by the another operation unit, the engagement state of the third rotation member and the fourth rotation member and the non-engagement state of the third rotation member and the fourth rotation member can be appropriately switched.
(4) The power transmission device according to (3), in which
   the another operation unit includes:
   another actuation element (retainer 282, guide 284) configured to move the another engagement element; and
   another operation element (operation rod 241, pin 283) provided to be capable of operating the another engagement element via the another actuation element or capable of operating the another engagement element without the another actuation element.
   According to (4), by controlling the another actuation element by the another operation element, the engagement state of the third rotation member and the fourth rotation member and the non-engagement state of the third rotation member and the fourth rotation member can be appropriately switched.
(5) The power transmission device according to (4), in which
   the another operation element includes:
   another advance and retraction element (pin 283) provided to be capable of advancing and retracting along an orthogonal direction orthogonal to the rotation axes; and
   another extending portion (operation rod 241) extending along the rotation axes and provided to be capable of advancing and retracting along the rotation axes.
   According to (5), by controlling the another advance and retraction element by the another extending portion, the another advance and retraction element can advance and retract along the orthogonal direction orthogonal to the rotation axes of the third rotation member and the fourth rotation member.
(6) The power transmission device according to (5), in which
   the second rotation member and the fourth rotation member are provided to be integrally rotatable, and
   the extending portion and the another extending portion are integrally provided, and are disposed at different positions in a direction of the rotation axes.
   According to (6), the second rotation member and the fourth rotation member can share a drive source, and the control can be simplified.
(7) The power transmission device according to (5) or (6), in which
   the another advance and retraction element is provided such that an inner end, which is an end portion of the another advance and retraction element on a side of the rotation axes in the radial direction, abuts on the another extending portion.
   According to (7), the inner end of the another advance and retraction element abuts on the another extending portion of the another operation element, and thus the another advance and retraction element can advance and retract along the orthogonal direction orthogonal to the rotation axes of the third rotation member and the fourth rotation member.
(8) The power transmission device according to any one of (3) to (7), in which
   the third rotation member is formed integrally with the second rotation element or mechanically connected to the second rotation element in an integrally rotatable manner.
   According to (8), the rotation speed-changed by the transmission unit is transmitted to the third rotation member.
(9) The power transmission device according to any one of (1) to (8), in which
   the transmission includes another transmission unit (first planetary mechanism P1) including:
      another first rotation element (sun gear SG1);
      another second rotation element (carrier PC1) provided to be capable of transmitting rotation with the another first rotation element; and
      another third rotation element (ring gear RG1) provided to be capable of transmitting rotation with the another second rotation element, and
   the another second rotation element of the another transmission unit is formed integrally with the first rotation element of the transmission unit or mechanically connected to the first rotation element of the transmission unit in an integrally rotatable manner.
   According to (9), the rotation speed-changed by the another transmission unit is transmitted to the transmission unit.
(10) A power transmission device (power transmission devices TM1 to TM4) including:
   a connection and disconnection mechanism (connection and disconnection mechanism 20); and
   a transmission (transmission T), in which
   the connection and disconnection mechanism includes:
      an engagement element (roller 281) disposed between a first rotation member (sun gear SG2) and a second rotation member (second shaft 182); and
      an operation unit (operation rod 241, pin 283, retainer 282, and guide 284) configured to operate the engagement element to an engagement state where the first rotation member and the second rotation member are integrally rotatable and a non-engagement state where the first rotation member and the second rotation member are relatively rotatable,
   the operation unit includes an actuation element (retainer 282, guide 284) configured to move the engagement element, and an operation element (operation rod 241, pin 283) provided to be capable of operating the engagement element via the actuation element or capable of operating the engagement element not via the actuation element,
   the first rotation member and the second rotation member are disposed such that respective rotation axes (rotation axis L1) of the first rotation member and the second rotation member coincide with each other, and at least a part of the first rotation member and the second rotation member overlap each other as viewed in a radial direction with respect to the rotation axes,
   the operation element includes:
      an advance and retraction element (pin 283) provided to be capable of advancing and retracting along the radial direction; and
      an extending portion (operation rod 241) extending along the rotation axes and provided to be capable of advancing and retracting along the rotation axes,
   the transmission includes a planetary transmission unit (second planetary mechanism P2) including:
      a first rotation element (sun gear SG2);
      a third rotation element (ring gear RG2);
      a fourth rotation element (planetary gear PG2) disposed to be capable of transmitting power between the first rotation element and the third rotation element; and
      a second rotation element (carrier PC2) supporting the fourth rotation element in a rotatable and revolvable manner,
   the planetary transmission unit is configured such that rotation speeds of three rotation elements including the first rotation element, the third rotation element and the second rotation element satisfy a collinear relationship in which the rotation speeds are aligned on a single straight line in a collinear diagram, and
   in the planetary transmission unit, a rotation axis of the third rotation element corresponds to the rotation axes of the first rotation member and the second rotation member.
   According to (10), the transmission can be disposed coaxially with the first rotation member and the second rotation member, and the power transmission device can be reduced in size.
(11) the power transmission device according to (10), in which
   power of a power source (motor M) is input to the first rotation element of the planetary transmission unit, and
   the power source includes an electric motor having a rotation axis (rotation axis L2) corresponding to the rotation axes of the first rotation member and the second rotation member.
   According to (11), the power source can also be disposed coaxially with the first rotation member and the second rotation member.
(12) A joint device (electric prosthetic leg 1) including:
   a first member (below-knee member 110);
   a second member (above-knee member 120);
   a coupling unit (knee joint mechanism 130) configured to couple the first member and the second member such that a formed angle formed between the first member and the second member is variable; and
   an enlarging and reducing device (enlarging and reducing device 200) capable of enlarging and reducing the formed angle formed between the first member and the second member, in which
   the enlarging and reducing device includes the power transmission device (power transmission devices TM1 to TM3) according to any one of (1) to (10).
   According to (12), the joint device can be reduced in size.
(13) A joint device (electric prosthetic leg 1) including:
   a first member (below-knee member 110);
   a second member (above-knee member 120);
   a coupling unit (knee joint mechanism 130) configured to couple the first member and the second member such that a formed angle formed between the first member and the second member is variable; and
   an enlarging and reducing device (enlarging and reducing device 200) capable of enlarging and reducing the formed angle formed between the first member and the second member, in which
   the enlarging and reducing device includes a power source (motor M), and a power transmission device (power transmission devices TM1 to TM4) configured to transmit power of the power source,
   the power transmission device has:
      a first power transmission path for transmitting the power at a first transmission ratio; and
      a second power transmission path for transmitting the power at a second transmission ratio different from the first transmission ratio,
   the enlarging and reducing device includes:
      a first connection and disconnection mechanism (first connection and disconnection mechanism 210) configured to switch between disconnection and connection of power in the first power transmission path; and
      a second connection and disconnection mechanism (second connection and disconnection mechanism 220) configured to switch between disconnection and connection of power in the second power transmission path,
   the first power transmission path includes a planetary transmission unit (first planetary mechanism P1), and
   the second power transmission path includes the planetary transmission unit and another planetary transmission unit (second planetary mechanism P2) different from the planetary transmission unit.
   According to (13), the transmission ratio can be increased while reducing the size, by performing speed change using the planetary transmission unit. Further, since the second power transmission path passes through the planetary transmission unit in the first power transmission path in addition to the another planetary transmission unit, the transmission ratio can be effectively increased.
(14) The joint device according to (13), in which
   each of the planetary transmission unit and the another planetary transmission unit includes a planetary transmission unit (first planetary mechanism P1, second planetary mechanism P2) configured such that rotation speeds of three of the rotation elements including a first rotation element (sun gear SG1, SG2), a second rotation element (carrier PC1, PC2), and a third rotation element (ring gear RG1, RG2) satisfy a collinear relationship in which the rotation speeds are aligned on a single straight line in a collinear diagram, and
   the another second rotation element of the another planetary transmission unit is formed integrally with the first rotation element of the planetary transmission unit or is mechanically connected to the first rotation element of the planetary transmission unit in an integrally rotatable manner.
   According to (14), the rotation speed-changed by the another planetary transmission unit can be transmitted to the planetary transmission unit.
(15) The joint device according to (14), in which
   the first connection and disconnection mechanism is disposed at a downstream side with respect to the planetary transmission unit in the first power transmission path, and
   the second connection and disconnection mechanism is disposed at a downstream side with respect to the planetary transmission unit and the another planetary transmission unit in the second power transmission path.
   According to (15), when the power is input from the downstream side, the number of accompanying members can be reduced.
(16) The joint device according to (15), in which
   when the first connection and disconnection mechanism is in a connection state and the second connection and disconnection mechanism is in a disconnection state, the power is output via the another planetary transmission unit and the first connection and disconnection mechanism, and
   when the first connection and disconnection mechanism is in a disconnection state and the second connection and disconnection mechanism is in a connection state, the power is output via the another planetary transmission unit, the planetary transmission unit, and the second connection and disconnection mechanism.

According to (16), by controlling the first connection and disconnection mechanism and the second connection and disconnection mechanism, the first power transmission path and the second power transmission path can be appropriately switched.

The present application is based on a Japan Patent Application (Patent Application No. 2023-058069) filed on March 31, 2023, and the contents thereof are incorporated herein by reference.

### REFERENCE SIGNS LIST

1 electric prosthetic leg (joint device)
20 connection and disconnection mechanism
110 below-knee member (first member)
120 above-knee member (second member)
130 knee joint mechanism (coupling unit)
182 second shaft (second rotation member, fourth rotation member)
200 enlarging and reducing device
210 first connection and disconnection mechanism (connection and disconnection mechanism)
220 second connection and disconnection mechanism (connection and disconnection mechanism)
241 operation rod (extending portion, another extending portion, operation element, another operation element, operation unit)
281 roller (engagement element)
282 retainer (actuation element, another actuation element, operation unit)
283 pin (advance and retraction element, other advance and retraction element, operation element, another operation element, operation unit)
284 guide (actuation element, another actuation element, operation unit)
TM1 to TM4 power transmission device
P1 first planetary mechanism (another transmission unit, another planetary transmission unit)
P2 second planetary mechanism (transmission unit, planetary transmission unit)
SG1 sun gear (another first rotation element)
SG2 sun gear (first rotation element)
RG1 ring gear (another third rotation element)
RG2 ring gear (third rotation element)
PC1 carrier (another second rotation element)
PC1a tubular portion (first rotation member)
PC2 carrier (second rotation element)
PC2a tubular portion (third rotation member)
PG2 planetary gear (fourth rotation element)
M motor (power source)
L1 rotation axis
L2 rotation axis
T transmission

## Claims

1. A power transmission device comprising:
a connection and disconnection mechanism; and
a transmission, wherein
the connection and disconnection mechanism includes:
an engagement element disposed between a first rotation member and a second rotation member; and
an operation unit configured to operate the engagement element to an engagement state where the first rotation member and the second rotation member are integrally rotatable and a non-engagement state where the first rotation member and the second rotation member are relatively rotatable,
the operation unit includes an actuation element configured to move the engagement element, and an operation element provided to be capable of operating the engagement element via the actuation element or capable of operating the engagement element not via the actuation element,
the first rotation member and the second rotation member are disposed such that respective rotation axes of the first rotation member and the second rotation member coincide with each other, and at least a part of the first rotation member and the second rotation member overlap each other as viewed in a radial direction with respect to the rotation axes,
the operation element includes:
an advance and retraction element provided to be capable of advancing and retracting along the radial direction; and
an extending portion extending along the rotation axes and provided to be capable of advancing and retracting along the rotation axes,
the transmission includes a transmission unit including:
a first rotation element configured by the first rotation member or a rotation member rotating integrally with the first rotation member;
a second rotation element provided to be capable of transmitting rotation with the first rotation element; and
a third rotation element provided to be capable of transmitting rotation with the second rotation element,
the second rotation element and the third rotation element are:
disposed outward than the second rotation member in the radial direction; and
disposed such that at least a part of the second rotation element and the third rotation element overlaps the second rotation member as viewed in the radial direction.

2. The power transmission device according to claim 1, wherein
the advance and retraction element is provided such that an inner end, which is an end portion of the advance and retraction element on a side of the rotation axes in the radial direction, abuts on the extending portion.

3. The power transmission device according to claim 1 or 2, further comprising:
a third rotation member and a fourth rotation member disposed such that the rotation axes and rotation axes of the third rotation member and the fourth rotation member coincide with each other, wherein
the connection and disconnection mechanism further includes:
another engagement element disposed between the third rotation member and the fourth rotation member; and
another operation unit configured to operate the another engagement element to an engagement state where the third rotation member and the fourth rotation member are integrally rotatable and a non-engagement state where the third rotation member and the fourth rotation member are relatively rotatable.

4. The power transmission device according to claim 3, wherein
the another operation unit includes:
another actuation element configured to move the another engagement element; and
another operation element provided to be capable of operating the another engagement element via the another actuation element or capable of operating the another engagement element not via the another actuation element.

5. The power transmission device according to claim 4, wherein
the another operation element includes:
another advance and retraction element provided to be capable of advancing and retracting along an orthogonal direction orthogonal to the rotation axes; and
another extending portion extending along the rotation axes and provided to be capable of advancing and retracting along the rotation axes.

6. The power transmission device according to claim 5, wherein
the second rotation member and the fourth rotation member are provided to be integrally rotatable, and
the extending portion and the another extending portion are integrally provided, and are disposed at different positions in a direction of the rotation axes.

7. The power transmission device according to claim 5 or 6, wherein
the another advance and retraction element is provided such that an inner end, which is an end portion of the another advance and retraction element on a side of the rotation axes in the radial direction, abuts on the another extending portion.

8. The power transmission device according to any one of claims 3 to 7, wherein
the third rotation member is formed integrally with the second rotation element or mechanically connected to the second rotation element in an integrally rotatable manner.

9. The power transmission device according to any one of claims 1 to 8, wherein
the transmission includes another transmission unit including:
another first rotation element;
another second rotation element provided to be capable of transmitting rotation with the another first rotation element; and
another third rotation element provided to be capable of transmitting rotation with the another second rotation element, and
the another second rotation element of the another transmission unit is formed integrally with the first rotation element of the transmission unit or mechanically connected to the first rotation element of the transmission unit in an integrally rotatable manner.

10. A power transmission device comprising:
a connection and disconnection mechanism; and
a transmission, wherein
the connection and disconnection mechanism includes:
an engagement element disposed between a first rotation member and a second rotation member; and
an operation unit configured to operate the engagement element to an engagement state where the first rotation member and the second rotation member are integrally rotatable and a non-engagement state where the first rotation member and the second rotation member are relatively rotatable,
the operation unit includes an actuation element configured to move the engagement element, and an operation element provided to be capable of operating the engagement element via the actuation element or capable of operating the engagement element not via the actuation element,
the first rotation member and the second rotation member are disposed such that respective rotation axes of the first rotation member and the second rotation member coincide with each other, and at least a part of the first rotation member and the second rotation member overlap each other as viewed in a radial direction with respect to the rotation axes,
the operation element includes:
an advance and retraction element provided to be capable of advancing and retracting along the radial direction; and
an extending portion extending along the rotation axes and provided to be capable of advancing and retracting along the rotation axes,
the transmission includes a planetary transmission unit including:
a first rotation element;
a third rotation element;
a fourth rotation element disposed to be capable of transmitting power between the first rotation element and the third rotation element; and
a second rotation element supporting the fourth rotation element in a rotatable and revolvable manner,
the planetary transmission unit is configured such that rotation speeds of three rotation elements including the first rotation element, the third rotation element and the second rotation element satisfy a collinear relationship in which the rotation speeds are aligned on a single straight line in a collinear diagram, and
in the planetary transmission unit, a rotation axis of the third rotation element corresponds to the rotation axes of the first rotation member and the second rotation member.

11. The power transmission device according to claim 10, wherein
power of a power source is input to the first rotation element of the planetary transmission unit, and
the power source includes an electric motor having a rotation axis corresponding to the rotation axes of the first rotation member and the second rotation member.

12. A joint device comprising:
a first member;
a second member;
a coupling unit configured to couple the first member and the second member such that a formed angle formed between the first member and the second member is variable; and
an enlarging and reducing device capable of enlarging and reducing the formed angle formed between the first member and the second member, wherein
the enlarging and reducing device includes the power transmission device according to any one of claims 1 to 10.

13. A joint device comprising:
a first member;
a second member;
a coupling unit configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission device configured to transmit power of the power source,
the power transmission device has:
a first power transmission path for transmitting the power at a first transmission ratio; and
a second power transmission path for transmitting the power at a second transmission ratio different from the first transmission ratio,
the enlarging and reducing device includes:
a first connection and disconnection mechanism configured to switch between disconnection and connection of power in the first power transmission path; and
a second connection and disconnection mechanism configured to switch between disconnection and connection of power in the second power transmission path,
the first power transmission path includes a planetary transmission unit, and
the second power transmission path includes the planetary transmission unit and another planetary transmission unit different from the planetary transmission unit.

14. The joint device according to claim 13, wherein
each of the planetary transmission unit and the another planetary transmission unit includes a planetary transmission unit configured such that rotation speeds of three rotation elements including a first rotation element, a second rotation element, and a third rotation element satisfy a collinear relationship in which the rotation speeds are aligned on a single straight line in a collinear diagram, and
the another second rotation element of the another planetary transmission unit is formed integrally with the first rotation element of the planetary transmission unit or is mechanically connected to the first rotation element of the planetary transmission unit in an integrally rotatable manner.

15. The joint device according to claim 14, wherein
the first connection and disconnection mechanism is disposed at a downstream side with respect to the planetary transmission unit in the first power transmission path, and
the second connection and disconnection mechanism is disposed at a downstream side with respect to the planetary transmission unit and the another planetary transmission unit in the second power transmission path.

16. The joint device according to claim 15, wherein
when the first connection and disconnection mechanism is in a connection state and the second connection and disconnection mechanism is in a disconnection state, the power is output via the another planetary transmission unit and the first connection and disconnection mechanism, and
when the first connection and disconnection mechanism is in a disconnection state and the second connection and disconnection mechanism is in a connection state, the power is output via the another planetary transmission unit, the planetary transmission unit, and the second connection and disconnection mechanism.
